# FASCICULE DE BREVET EUROPEEN

(11) **EP 4 043 005 B1**
(45) Date de publication et mention de la délivrance du brevet: **20.12.2023**
(21) Numéro de dépôt: 21305186.5
(22) Date de dépôt: 12.02.2021
(51) Int. Cl.: A61K 8/99, A61K 8/9789, A61Q 19/00

(54) **COMPOSITION COSMÉTIQUE COMPRENANT UN HYDROLYSAT DE LEVURE**
KOSMETISCHE ZUSAMMENSETZUNG, DIE EIN HEFEHYDROLYSAT ENTHÄLT
COSMETIC COMPOSITION COMPRISING A YEAST HYDROLYSATE

(43) Date de publication de la demande: 17.08.2022
(73) Titulaire: Chanel Parfums Beauté, 92200 Neuilly-sur-Seine (FR)
(72) Inventeur: FUZZATI, Nicola, 93645 PANTIN CEDEX (FR)
(74) Mandataire: Plasseraud IP

(56) Documents cités:
- FR-A1- 2 927 254
- FR-A1- 2 976 490
- KR-A- 20160 093 427
- KR-A- 20170 126 142

## Description

### Domaine technique

La présente invention a pour objet une composition cosmétique comprenant un hydrolysat de levure de l'espèce *Pichia naganishii* obtenu à partir d'un exsudat de *Camellia japonica,* ainsi que son utilisation en cosmétique, pour favoriser l'hydratation de la peau et/ou protéger la peau humaine contre le dessèchement et/ou améliorer la fonction barrière et/ou pour prévenir/réduire le vieillissement cutané.

### Etat de l'art

La peau est principalement constituée de trois couches, à savoir, en partant de la plus superficielle, l'épiderme, le derme et l'hypoderme.

L'épiderme est en particulier constitué de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules mortes totalement kératinisées sous forme d'enveloppes cornées constituées de protéines et de lipides tels que des céramides. Lors de ce processus de différenciation, des lipides épidermiques intercornéocytaires sont en outre formés puis organisés sous forme de bicouches (feuillets) dans le stratum corneum. Ils participent, avec les enveloppes cornées précitées, à la fonction barrière de l'épiderme.

La fonction barrière de l'épiderme peut toutefois se trouver perturbée dans certaines conditions climatiques (sous l'effet du froid et/ou du vent, par exemple), ou encore sous l'effet du stress ou de la fatigue, notamment, favorisant ainsi la pénétration d'allergènes, d'agents irritants ou de micro-organismes qui occasionnent ainsi un dessèchement cutané susceptible de générer des sensations d'inconfort telles que des tiraillements ou des rougeurs, et également d'altérer l'éclat du teint et la souplesse de la peau.

Pour prévenir ce phénomène ou le corriger, il est connu d'appliquer sur la peau des compositions cosmétiques renfermant des agents hygroscopiques, tels que des sucres ou des polyols, destinés à capter l'eau présente dans la peau et à freiner ainsi son évaporation. Par ailleurs, ces compositions incorporent fréquemment des actifs agissant sur une ou plusieurs des différentes cibles biologiques intervenant soit dans les processus de régénération de la peau, en particulier dans la différenciation des kératinocytes, la synthèse des lipides épidermiques et la cohésion des cornéocytes, soit dans la synthèse endogène de constituants du facteur d'hydratation naturel (NMF, Natural Moisturizing Factor) de la peau, en particulier dans la synthèse de protéoglycanes.

Les protéoglycanes jouent d'ailleurs un rôle important dans le maintien de l'hydratation et dans le raffermissement de la peau et tout particulièrement, l'acide hyaluronique. Cependant, avec l'âge, l'acide hyaluronique diminue en quantité et en qualité, entraînant un dessèchement et un relâchement de la peau, la perte de son élasticité ce qui entraine donc l'apparition de rides. Il est donc nécessaire de trouver des actifs capables de stimuler sa production.

Des exemples de tels actifs sont notamment les α- et β-hydroxyacides, notamment l'acide lactique, l'acide glycolique et l'acide salicylique, l'urée, ou les composés aminosulfoniques.

Toutefois, il subsiste toujours le besoin de proposer de nouveaux actifs cosmétiques permettant une hydratation et une prévention anti-âge plus efficace de la peau.

En outre, compte tenu de la recherche toujours croissante par les consommateurs de produits naturels renfermant le moins d'ingrédients synthétiques possibles, et des contraintes réglementaires de plus en plus lourdes pesant sur les composés issus de l'industrie chimique, il serait souhaitable que ces actifs cosmétiques soient d'origine naturelle,

Les documents FR 2 976 490 A1, FR 2 927 254 A1, KR 2016 0093427 A et KR 2017 0126142 proposent des utilisations cosmétiques de la levure du genre Pichia.

### Résumé de l'invention

C'est l'objectif de la présente invention qui, à cet effet, propose une nouvelle composition cosmétique comprenant un hydrolysat de levure de l'espèce *Pichia naganishii.*
La levure *Pichia naganishii,* également dénommée *Ogataea naganishii* peut être isolée à partir d'un exsudat du Camélia du Japon ou *Camellia japonica.* Cette levure spécifique a été identifiée jusqu'à présent uniquement sur deux plantes, le *Camellia japonica* et *Sakaki ochnacea* et dans une seule zone géographique située au Japon. Une souche de la levure *Pichia naganishii* peut également être obtenue auprès de la Banque ATCC (American Type Culture Collection) sous le numéro 32418.

Il est connu de l'art antérieur, des brevets portant sur la production de molécules par la levure *Pichia naganishii* telles que l'acide glycolique, le dérivé de chioropropanediol, l'hydroxy-ketoester, le propanol, la muscone et la glycolate, mais aucun art antérieur ne décrit une utilisation cosmétique ou un principe actif issu de cette levure.

De manière surprenante, les inventeurs ont démontré que l'application d'hydrolysats de *Pichia naganishii* sur des cultures de kératinocytes permet de stimuler la production d'acide hyaluronique, ainsi que la synthèse de plusieurs protéines impliquées dans la fonction barrière et l'hydratation de la peau. Ces hydrolysats peuvent donc être utilisés pour prévenir/ralentir le vieillissement cutané ainsi que pour améliorer l'hydratation et la fonction barrière de la peau.

L'invention concerne donc, selon un premier aspect, une composition comprenant un hydrolysat de la biomasse de la levure *Pichia naganishii.* En particulier, l'invention concerne une composition comprenant au moins un hydrolysat de levure de l'espèce *Pichia naganishii,* laquelle levure étant obtenue à partir d'un exsudat de *Camellia japonica.*

L'invention se rapporte également à une composition comprenant au moins un hydrolysat de levure de l'espèce *Pichia naganishii* obtenu selon le procédé de préparation comprenant notamment une solubilisation de la levure *Pichia naganishii* dans l'eau, une hydrolyse enzymatique, une séparation de la phase soluble et insoluble et récupération de la phase soluble, une inactivation enzymatique, et éventuellement une concentration de l'hydrolysat suivi d'une filtration stérilisante.

L'hydrolysat de *Pichia naganishii* est d'origine naturelle et présente avantageusement un effet hydratant et fonction barrière sans l'agresser ainsi qu'un effet anti-âge. Il répond ainsi aux problématiques de l'art antérieur. Il peut être utilisé pour des applications cosmétiques, préférentiellement sous une forme adaptée à une application topique. La composition cosmétique comprend au moins 0,1% en poids de l'hydrolysat de *Pichia naganishii.*

L'invention concerne, selon un deuxième aspect, une utilisation cosmétique non thérapeutique d'un hydrolysat de *Pichia naganishii* ou de la composition en contenant, pour améliorer l'hydratation et/ou protéger la peau humaine contre le dessèchement et/ou améliorer la fonction barrière et/ou pour prévenir/réduire le vieillissement cutané.

D'autres caractéristiques et avantages ressortiront de la description détaillée de l'invention et des exemples qui vont suivre.

### Description détaillée de l'invention

### Définitions

Par « hydrolysat de *Pichia naganishii* », on entend tout principe actif issu de la levure *Pichia naganishii.* Cet hydrolysat *est le produit* obtenu par un procédé comprenant au moins une étape d'hydrolyse de *Pichia naganishii.* Le terme hydrolysat de *Pichia naganishii* exclut les molécules produites uniquement par fermentation de *Pichia naganishii.*

Par « *Pichia naganishii* », on entend toute levure de la famille des *Saccharomycetaceae,* du genre *Pichia* et de l'espèce *Pichia naganishii.* Une souche de la levure *Pichia naganishii* a été enregistrée dans une collection de levures, sous le numéro ATCC 32418. Elle est également connue sous le nom : *Ogataea naganishii. Pichia naganishii* peut être isolée à partir d'un exsudat du *Camellia japonica.*

Un « exsudat de *Camellia japonica* » est une substance excrétée par la plante *Camellia japonica.*

### Hydrolysat de Pichia naganishii

La présente invention a donc pour objet une composition cosmétique comprenant au moins un hydrolysat de levure de l'espèce *Pichia naganishii.*

Selon un mode de réalisation préféré, la levure *Pichia naganishii* peut être obtenue à partir d'un exsudat de *Camellia japonica* ou à partir de la souche déposée sous le numéro ATCC 32418.L'hydrolysat peut notamment comprendre des protéines et/ou des carbohydrates. Lorsque l'hydrolysat comprend des protéines, il comprend préférentiellement entre 20% et 60% de protéines, en poids de matière sèche de l'hydrolysat.

Selon un mode de réalisation, l'hydrolysat comprend entre 5% et 25% de carbohydrates, plus préférentiellement 12% en poids de matière sèche de l'hydrolysat.

Préférentiellement, le taux de cendres est compris entre 5 et 35% en poids, encore plus préférentiellement compris entre 20 et 30%.

La teneur en cendres brutes peut être déterminée par la pesée des résidus issus de l'incinération des échantillons de l'hydrolysat à 550°C dans un four à moufle électrique.

L'hydrolysat est préférentiellement obtenu par une hydrolyse enzymatique. Ainsi, l'hydrolysat est préférentiellement un hydrolysat enzymatique. A titre d'exemple, les enzymes utilisées pour l'hydrolyse peuvent être une protéase ou une carbohydrase.

L'hydrolysat de *Pichia naganishii* peut se présenter sous forme solide ou sous forme liquide.

Lorsqu'il se présente sous forme liquide, l'hydrolysat se présente sous forme d'un liquide limpide, de couleur jaune clair à l'odeur caractéristique.

Lorsqu'il se présente sous forme solide l'hydrolysat est associé à un support choisi parmi la maltodextrine, la gomme arabique, la lécithine de soja ou l'isomalt. Selon un mode de réalisation particulièrement adapté, l'ensemble hydrolysat + support est constitué par au moins 10% en poids d'hydrolysat et au plus 90% en poids de support.

Dans le cas d'une forme solide dans laquelle le l'hydrolysat est associé à un support, les teneurs en protéines, en sucres et en cendres annoncés ci-dessus sont modifiées, le support étant généralement constitué majoritairement de sucres.

L'hydrolysat de *Pichia naganishii* peut éventuellement être intégré dans une composition cosmétique, notamment une composition comprenant au moins 0,1% en poids dudit hydrolysat. En particulier, la composition est sous une forme adaptée à une application topique telle qu'une crème ou une lotion.

### Procédé d'extraction

L'hydrolysat de *Pichia naganishii* peut être obtenu par tout procédé comprenant au moins une étape d'hydrolyse de *Pichia naganishii.* De façon particulièrement préférée, il est obtenu par un procédé comprenant une étape d'hydrolyse enzymatique.

Préalablement au procédé d'obtention de l'hydrolysat en tant que tel, il convient de produire la biomasse de *Pichia naganishii.* Cette étape est réalisée selon le mode de culture des levures dans un milieu adapté à leur développement, de manière classique pour l'homme de métier. Une fois la biomasse obtenue, on réalise une hydrolyse, en vue d'obtenir des molécules actives.

Selon un mode de réalisation particulièrement adapté, l'hydrolysat de *Pichia naganishii* est obtenu par la mise en oeuvre des étapes suivantes :
- solubilisation à raison d'au moins 40 g/L de la levure *Pichia naganishii* dans l'eau,
- hydrolyse, préférentiellement hydrolyse enzymatique,
- séparation de la phase soluble et insoluble et récupération de la phase soluble,
- inactivation enzymatique, et éventuellement filtration,
- éventuellement concentration et filtration stérilisante.

Les conditions d'hydrolyse sont choisies pour hydrolyser à la fois les parois des levures et le milieu intracellulaire des levures, préférentiellement l'hydrolyse est réalisée par voie enzymatique.

La séparation des phases soluble et insoluble est réalisée par tout moyen par exemple par centrifugation, filtration ou décantation. Préférentiellement la séparation des phases soluble et insoluble est réalisée par centrifugation, permettant ainsi la récupération de la phase soluble contenant entre autres les protéines solubles.

L'inactivation de l'enzyme est préférentiellement réalisée par traitement thermique. Cette inactivation est alors réalisée selon les préconisations du fournisseur de l'enzyme.

Eventuellement, le procédé comprend une étape de filtration après la récupération de la phase soluble pour éliminer les particules encore en suspension. Ainsi, cette étape de filtration permet la purification de la phase soluble récupérée et est réalisée afin d'éliminer les molécules de haut poids moléculaire (les enzymes et les polymères...) .

L'hydrolysat obtenu à ce stade peut éventuellement être encore concentré et/ou purifié, préférentiellement par des étapes d'ultrafiltrations successives à travers des filtres de porosité différentes, en conservant les filtrats à chaque étape et/ou par une méthode de type chromatographique.

L'hydrolysat obtenu après hydrolyse et filtration, avant ou après concentration et filtration stérilisante, est un hydrolysat de *Pichia naganishii* se présentant sous forme liquide.

L'hydrolysat obtenu peut ensuite être séché et associé à un support, pour se présenter sous forme solide. Cette phase peut être réalisée par la mise en oeuvre des étapes suivantes :
- un support d'atomisation, de préférence la maltodextrine, est ajouté dans l'hydrolysat de *Pichia naganishii,* jusqu'à 90% (en masse/volume) ;
- cette solution est ensuite concentrée sous vide ;
- une élimination des bactéries éventuellement présentes est réalisée par traitement thermique ;
- l'atomisation permet d'obtenir une poudre.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

### Composition cosmétique

La présente invention a pour objet une composition cosmétique comprenant, dans un milieu physiologiquement acceptable, au moins un hydrolysat de *Pichia naganishii.*

La composition mise en oeuvre selon l'invention comprend généralement, outre l'hydrolysat décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements).

Elle peut être avantageusement appliquée sur la peau du visage, du cou, des mains et éventuellement du décolleté ou en variante sur une partie quelconque du corps. La composition renfermant cet hydrolysat peut être appliquée le matin et/ou le soir, sur l'ensemble du visage, du cou, des mains et éventuellement du décolleté voire du corps.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau-dans-huile ou huile-dans-eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'hydrolysat de *Pichia naganishii :*
- au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- un ou plusieurs agent(s) humectants, tels que les polyols (glycérine, diglycérine, le propanediol, le caprylyl glycol, le pentylène glycol, l'hexanediol), les sucres, les glycosaminoglycanes tels que l'acide hyaluronique et ses sels et esters; et les polyquaterniums tel que le lipidure PMB. Ledit agent humectant sera présent dans la composition à une teneur de l'ordre de 0 à 30%, de préférence 0.005 à 10% en poids total de la composition.
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglycéride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycérides) , les huiles végétales, le phytosqualane, les alcanes (undecane, tridecane). Ledit agent émollient sera présent dans la composition à une teneur de l'ordre de 0,1 à 30%, de préférence 0,5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP). Ledit agent gélifiant et/ou épaississant sera présent dans la composition à une teneur de l'ordre de 0,1 à 10% en poids total de la composition.
- Un ou plusieurs agent(s) tensioactif(s), tels que les lecithines, les dérivés de polyglycérol, les dérivés de sucre (les dérivés de glucosides ou de xylosides commercialisés sous le nom MONTANOV 68, MONTANOV 202, Montanov 82, MONTANOV L, EASYNOV), les phosphates (C20-22 alkyl phosphate commercialisé sous le nom SENSANOV WR). Ledit agent tensioactif, sera présent dans une teneur de l'ordre de 0,1 à 8%, de préférence 0,5 à 3% en poids, par rapport au poids total de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 commercialisés sous le nom NP RIGIN, MATRIXYL 3000, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc. Ledit agent actif sera présent dans la composition à une teneur de l'ordre de 0,1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

### Utilisation cosmétique

La composition cosmétique selon l'invention comprend un hydrolysat de *Pichia naganishii* particulièrement efficace pour hydrater la peau et renforcer la fonction barrière. En particulier, lorsqu'il est appliqué sur la peau permet de :
- maintenir l'hydratation,
- renforcer la fonction barrière.
- protéger la peau humaine contre le dessèchement
- renforcer les propriétés biomécaniques de la peau
- atténuer l'apparition des rides.
Avantageusement, la composition cosmétique selon l'invention comprend un hydrolysat de *Pichia naganishii* capable de stimuler la synthèse d'acide hyaluronique dans des kératinocytes humains et ainsi promouvoir une action hydratante et anti-âge.

La composition cosmétique selon l'invention comprend un hydrolysat de *Pichia naganishii* permettant également d'augmenter l'expression de l'hyaluronane synthase 2, de l'aquaporine 3, de la caspase 14, de la filaggrine, de la desmogléine-1, de la loricrine, afin d'améliorer l'hydratation de la peau en maintenant l'homéostasie de la fonction barrière.

L'invention vise ainsi une utilisation cosmétique, non thérapeutique, d'un hydrolysat de *Pichia naganishii* pour ses effets cosmétiques, seul ou dans une composition, en particulier pour au moins un effet cosmétique choisi parmi l'amélioration de l'hydratation de la peau, un renforcement de la barrière cutanée et la prévention/réduction du vieillissement cutané.

Par utilisation non thérapeutique, on entend au sens de l'invention une utilisation cosmétique de l'hydrolysat de *Pichia naganishii* ou d'une composition le comprenant, destinée à des sujets sains non malades, en particulier à des sujets avec une peau saine.

L'invention est à présent illustrée par les exemples non limitatifs suivants.

### Exemples

### Exemple 1 - Principe actif (PA1)

L'hydrolysat de *Pichia naganishii* de l'exemple 1 (PA1) est obtenu par le procédé suivant :
- Solubilisation de P. *naganishii* dans l'eau, préférentiellement à raison de 50g/l,
- Hydrolyse enzymatique avec une carbohydrase,
- Séparation des phases solubles et insolubles,
- Inactivation enzymatique par traitement thermique,
- Filtration,
- Concentration,
- Filtration et filtration stérilisante.

L'hydrolysat de *Pichia naganishii* obtenu possède les caractéristiques suivantes : 45% de carbohydrates et 7% de protéines (en pourcentage par rapport à la matière sèche)

### Exemple 2 - Principe actif (PA2)

L'hydrolysat de *Pichia naganishii* de l'exemple 2 (PA2) est obtenu par le procédé suivant :
- Solubilisation de P. *naganishii* dans l'eau, préférentiellement à raison de 50g/l,
- Hydrolyse enzymatique avec une protéase,
- Séparation des phases solubles et insolubles,
- Inactivation enzymatique par traitement thermique,
- Filtration,
- Concentration,
- Filtration et filtration stérilisante.

L'hydrolysat de *Pichia naganishii* obtenu possède les caractéristiques suivantes : 12% de carbohydrates, 29% de cendres et 59% de protéines (en pourcentage par rapport à la matière sèche). L'hydrolysat est sous la forme d'une solution aqueuse liquide limpide, couleur jaune clair à l'odeur caractéristique.

### Exemple 3 - Principe actif (PA3)

L'hydrolysat de *Pichia naganishii* de l'exemple 3 (PA3) est obtenu par un procédé similaire à celui de l'exemple 2 et comprend une étape de filtration à l'aide de charbon.

L'hydrolysat de *Pichia naganishii* obtenu possède les caractéristiques suivantes : 45% de carbohydrates et 11% de protéines (en pourcentage par rapport à la matière sèche).

### Exemple 4 - Principe actif hors invention (PA4)

L'hydrolysat de l'exemple 4 (PA4) est obtenu par le procédé suivant :
- solubilisation de *Pichia anomala* dans l'eau, préférentiellement à raison de 20g/l,
- hydrolyse basique de la solution de *Pichia anomala*,
- séparation des phases soluble et insoluble, par exemple par filtration, centrifugation ou décantation,
- récupération de la phase soluble,
- filtration et purification pour récupérer les molécules,
- hydrolyse enzymatique des protéines, préférentiellement au moyen d'une protéase,
- filtration et purification pour récupérer les bio-peptides de taille inférieure à 5000Da.

### Exemple 5 - Effet de l'hydrolysat de Pichia naganishii sur la synthèse d'acide hyaluronique.

L'objectif de cet essai est de comparer l'effet de l'hydrolysat de *Pichia naganishii* (PA2) *in vitro* sur kératinocytes humains, sur la synthèse d'acide hyaluronique avec l'effet d'un principe actif hors invention (PA4).

Le protocole opératoire de l'étude est décrit en suivant.

Les kératinocytes humains normaux sont ensemencés puis incubés à 37°C dans une étuve contenant 5% de CO2. Puis le milieu de culture est éliminé et remplacé par du milieu contenant les principes actifs:
- PA2 à 1,00% (V/V)
- PA4 à 1,00% (V/V)

Les cellules sont ensuite incubées à 37°C dans une atmosphère contenant 5% de CO2 pendant 48 heures. Les surnageants sont récupérés avant d'être analysés par dosage ELISA.

Les résultats sont présentés dans le tableau 1 ci-après.

**[Tableau 1]**

| | **Synthèse d'acide hyaluronique (%)** | **Taux d'acide hyaluronique / Témoin (%)** |
|---|---|---|
| Témoin | 100 | |
| PA2 1,00% | 150 | + 50 |
| PA4 1,00% | 80 | - |

Ainsi, seul l'hydrolysat de *Pichia naganishii* (PA2) stimule la synthèse d'acide hyaluronique.

L'étude est également réalisée à différentes doses de principes actifs (hydrolysats) comme suit :
- PA1 à 0,25% et 1,00% (V/V)
- PA2 à 0,25% et 1,00% (V/V)
- PA3 à 0,25% et 1,00% (V/V)

Les résultats sont présentés dans le tableau 2 ci-après.

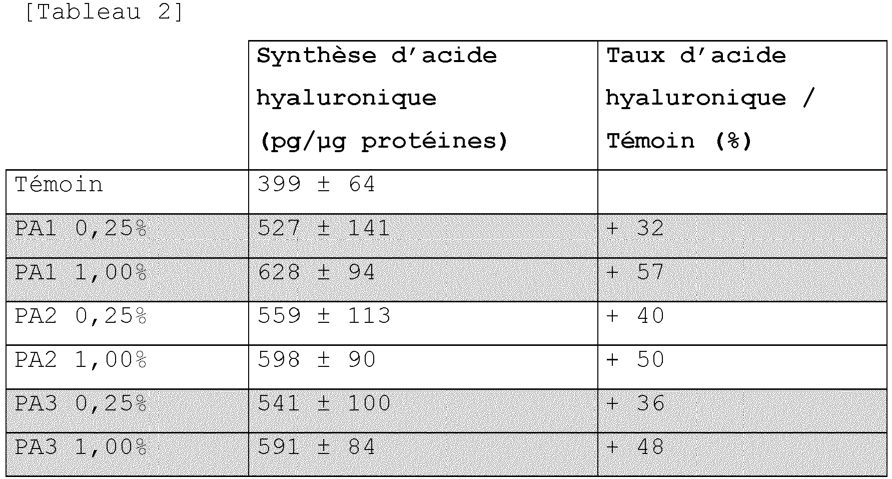

Ainsi, les hydrolysats de *Pichia naganishii* à 1% stimulent la synthèse de l'acide hyaluronique de 57% pour PA1, 50% pour PA2, 48% pour PA3. Comme expliqué ci-dessus, l'acide hyaluronique joue un rôle important dans le maintien de l'hydratation et dans l'élasticité de la peau. La diminution de la teneur en acide hyaluronique avec l'âge entraîne un dessèchement et un relâchement de la peau, ainsi qu'une perte de son élasticité, entraînant ainsi l'apparition de rides. Comme le sait l'homme du métier, les produits stimulant la synthèse d'acide hyaluronique dans la peau permettent de prévenir son dessèchement, d'améliorer son hydratation et de réduire/prévenir son vieillissement (et notamment l'apparition de rides). Les hydrolysats de *Pichia naganishii* peuvent donc être utilisés pour ces applications.

### Exemple 6 - Effet de l'hydrolysat de Pichia naganishii sur l'expression de HAS-2 et l'aquaporine 3.

L'objectif de cette étude est d'évaluer l'effet des hydrolysats de *Pichia naganishii* PA1, PA2 et PA3 sur leur capacité à augmenter l'expression des ARNm codant pour HAS-2 et l'aquaporine 3.

Cette étude a été réalisée sur kératinocytes humains normaux par PCR quantitative.

Le protocole opératoire de l'étude est décrit en suivant.

Les kératinocytes humains normaux sont ensemencés puis incubés à 37°C dans une étuve contenant 5% de CO2.

Puis le milieu de culture est éliminé et remplacé par du milieu contenant les principes actifs issus de *Pichia naganishii* :
- PA1 à 0,25% et 1,00% (V/V)
- PA2 à 0,25% et 1,00% (V/V)
- PA3 à 0,25% et 1,00% (V/V)

Les cellules sont ensuite incubées à 37°C dans une atmosphère contenant 5% de CO2. Les cellules sont récupérées et les ARN totaux extraits pour l'étude de HAS-2, de l'aquaporine 3, la caspase 14 et la filaggrine. Les ARN ont été reverses-transcripts et les ADN complémentaires obtenus ont été analysés par la technique de PCR quantitative.

Les ARNm de témoins de référence, ont été également analysés en parallèle des ARNm de l'aquaporine 3 et HAS-2.

La quantification de l'incorporation de fluorescence (SYBR Green) est mesurée en continu à l'aide d'un thermocycleur. L'analyse des Ct (quantification relative) est réalisée à l'aide d'un logiciel. Les résultats sont présentés dans le tableau 5 ci-après.

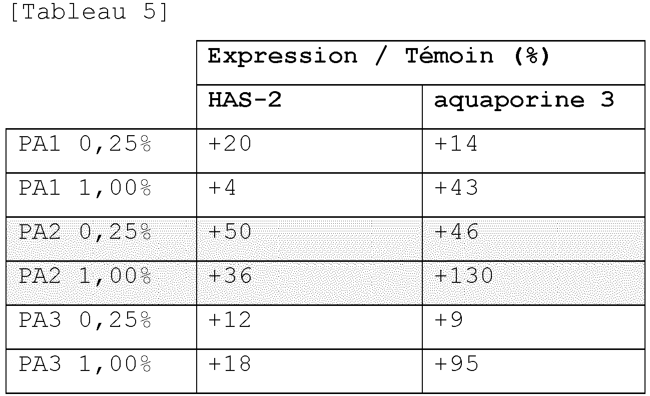

Ainsi, les hydrolysats de *Pichia naganishii* PA1, PA2 et PA3 augmentent l'expression de HAS-2 et de l'aquaporine 3, protéines connues pour leur action hydratante. Ces hydrolysats peuvent donc être utilisés afin d'améliorer l'hydratation de la peau.

### Exemple 7 - Effet de l'hydrolysat de Pichia naganishii sur les gènes de la barrière physique épidermique.

L'étude vise à évaluer l'effet de l'hydrolysat de *Pichia naganishii* PA2 sur sa capacité à augmenter l'expression des gènes de la barrière physique épidermique : desmogléine-1, fillaggrine, loricrine, caspase 14.

Cette étude a été réalisée sur kératinocytes humains normaux par PCR quantitative.

Le protocole opératoire de l'étude est décrit en suivant.

Les kératinocytes humains normaux sont ensemencés puis incubés à 37°C dans une étuve contenant 5% de CO2. Puis, le milieu de culture est éliminé et remplacé par du milieu contenant le produit issu de PA2 à 0,5%, 1,0% et 2,0% (V/V).

Les cellules sont ensuite incubées à 37°C dans une atmosphère contenant 5% de CO2. Les ARN totaux extraits ont été reverses-transcripts et les ADN complémentaires obtenus ont été analysés par la technique de PCR quantitative.

Les ARNm des gènes : desmogléine-1, filaggrine, loricrine, caspase 14, ont été analysés en parallèle des ARNm de témoins de référence pour la normalisation.

La fluorescence (SYBR Green) est mesurée en continu à l'aide d'un thermocycleur. L'analyse des Ct (quantification relative) est réalisée à l'aide d'un logiciel.

Les résultats sont présentés dans les tableaux 6 et 7 ci-après.

**[Tableau 6]**

| | **Expression / Témoin (%)** | | |
|---|---|---|---|
| | **PA2 0,5%** | **PA2 1,0%** | **PA2 2,0%** |
| Desmogléine-1 | 80 | 84 | 184 |
| Filaggrine | 33 | Va | 64 |
| Loricrine | 91 | Va | 89 |
| Caspase 14 | 95 | 150 | 370 |
| Va = valeurs aberrantes | | | |

Testé à 2,0%, l'hydrolysat de *Pichia naganishii* PA2 augmente significativement l'expression des gènes de la barrière physique épidermique. Il permet donc de renforcer la fonction barrière de la peau.

### Exemple 8 - Effet de l'hydrolysat de Pichia naganishii sur le réseau membranaire, en particulier sur les jonctions serrées.

Le but de cette étude est de visualiser l'effet des hydrolysats de *Pichia naganishii* sur le réseau membranaire formé par zonula occludens ZO-1, un des constituants majeurs des jonctions serrées.

L'étude a été réalisée par immunocytologie sur des kératinocytes humains normaux après agression au Sodium Lauryl Sulfate (SLS) à 0,4mM.

Le protocole opératoire de l'étude est décrit en suivant.

Les kératinocytes humains normaux sont ensemencés puis incubés à 37°C dans une étuve contenant 5% de CO2. Puis, les kératinocytes humains normaux sont traités avec une solution de CaCl2 à 1 mM pendant 48h afin de permettre la formation du réseau de jonctions serrées. Ensuite, les kératinocytes humains normaux sont traités avec une solution de SLS à 0,4 mM pendant 30 minutes.

A la fin de l'incubation, la solution de SLS est éliminée et remplacée par du milieu contenant les hydrolysats de *Pichia naganishii :*
- PA1 à 0,25% et 1,00% (V/V)
- PA2 à 0,25% et 1,00% (V/V)
- PA3 à 0,25% et 1,00% (V/V).

Les cellules sont incubées pendant 48h dans une étuve à 37°C en atmosphère humide contenant 5% de CO2.

Les cellules sont immunomarquées à l'aide d'un fluorophore. La visualisation est réalisée sur microscope couplé à un système d'analyse d'image. L'intensité du marquage des ZO-1 est proportionnelle à l'intensité de fluorescence verte présente au niveau membranaire des cellules. Plus la couleur verte est importante, plus le taux de synthèse des ZO-1 est élevé.

De plus, une analyse quantitative des images a été réalisée à l'aide du logiciel Matlab. Cette quantification est exprimée en Unité Arbitraire (UA).

Les résultats sont les suivants. Après agression SLS de kératinocytes humains, le réseau de ZO-1 membranaire induit par le CaCl2 est fortement altéré.

Testés à 0,25% puis à 1% sur kératinocytes humains agressés, les hydrolysats de *Pichia naganishii* augmentent respectivement la synthèse et la formation du réseau membranaire de ZO-1 de :
- 127% et 139% pour PA1
- 33% et 46% pour PA2
- 47% et 79% pour PA

Ainsi, les hydrolysats de *Pichia naganishii* augmentent l'expression des gènes impliqués dans la formation des jonctions serrées, permettant ainsi d'améliorer la fonction barrière et l'hydratation de la peau.

### Exemple 9 - Composition cosmétique

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

A - émulsion huile/eau gel

| **Nom INCI** | **(% W/W)** |
|---|---|
| LIMNANTHES ALBA (MEADOWFOAM) SEED OIL | 1-10 |
| BUTYROSPERMUM PARKII BUTTER (LIPEX SHEASOFT) | 1-10 |
| BUTYROSPERNUM PARKII BUTTER EXTRACT (LIPEX SHEA TRIS) | 1-10 |
| CAMELLIA OLEIFERA SEED OIL | 1-10 |
| CAPRYLIC/CAPRIC TRIGLYCERIDE | 1-10 |
| SQUALANE | 1-10 |
| AMMONIUM ACRYLOYLDIMETHYLTAURATE/VP COPOLYMER | 0.1-5 |
| ACRYLATES/C10-30 ALKYL ACRYLATE CROSSPOLYMER | 0.1-2 |
| XANTHAN GUM | 0.01-5 |
| TREMELLA FUCIFORMIS (MUSHROOM) EXTRACT | 0.01-5 |
| ORYZA SATIVA (RICE) POWDER | 0.1-5 |
| SODIUM HYALURONATE | 0.01-3 |
| GLYCERIN | 1-30 |
| POLYQUATERNIUM-51 | 1-10 |
| TOCOPHERYL ACETATE | 0.1-5 |
| NIACINAMIDE | 0.1-5 |
| SPHINGOMONAS FERMENT EXTRACT | 0.01-5 |
| POLIANTHES TUBEROSA | 0.01-5 |
| BETAINE | 0.1-10 |
| SODIUM PCA | 0.5-5 |
| SACCHARIDE ISOMERATE | 0.5-5 |
| HYDROLYSAT DE *PICHIA NAGANISHII* | 0.001-10 |
| YEAST EXTRACT | 0.1-5 |
| GLYCOLS (CAPRYLYL GLYCOL AND/OR PENTYLENE GLYCOL AND/OR BUTYLENE GLYCOL AND/OR PROPANEDIOL) | 0,1-10 |
| WATER | Qs 100 |

b - émulsion huile/eau crème

| **Nom INCI** | **(% w/w)** |
|---|---|
| JOJOBA ESTERS | 1-5 |
| LIMNANTHES ALBA (MEADOWFOAM) SEED OIL | 0.1-5 |
| CANOLA OIL | 1-10 |
| ARGANIA SPINOSA KERNEL OIL | 0.1-10 |
| MORINGA OIL/HYDROGENATED MORINGA OIL ESTERS | 1-10 |
| C8-12 ACID TRIGLYCERIDE | 1-5 |
| LAUROYL LYSINE | 1-5 |
| CAMELLIA OLEIFERA SEED OIL | 1-10 |
| PHYTOSTERYL/OCTYLDODECYL LAUROYL GLUTAMATE | 1-5 |
| SQUALANE | 1-10 |
| AMMONIUM ACRYLOYLDIMETHYLTAURATE/VP COPOLYMER | 1-5 |
| CETEARYL ALCOHOL & CETEARYL GLUCOSIDE | 1-7 |
| HYDROGENATED LECITHIN | 0.1-5 |
| CHONDRUS CRISPUS (CARRAGEENAN) | 0.1-5 |
| SCLEROTIUM GUM | 0,01-2 |
| CENTELLA ASIATICA LEAF EXTRACT | 0.1-5 |
| ADENOSINE | 0.1-0.5 |
| NIACINAMIDE | 0.1-5 |
| SECALE CEREALE (RYE) SEED EXTRACT | 0.1-5 |
| PALMITOYL TRIPEPTIDE-1 & PALMITOYL TETRAPEPTIDE-7 | 1-5 |
| PLANKTON EXTRACT | 0.1-5 |
| YEAST EXTRACT | 1-3 |
| HYDROLYSAT DE *PICHIA NAGANISHII* | 0.001-10 |
| GLYCYRRHIZA GLABRA EXTRACT | 0.001-5 |
| TRANEXAMIC CETYL ESTER | 0.001-5 |
| ASCORBYL GLUCOSIDE | 0.001-5 |
| WATER | Qs 100 |

Ces compositions peuvent être appliquées tous les jours, matin et/ou soir, sur la peau.

## Revendications

1. Composition cosmétique comprenant, dans un milieu physiologiquement acceptable, au moins un hydrolysat de *Pichia naganishii.*

2. Composition cosmétique selon la revendication précédente, **caractérisée en ce que** l'hydrolysat est un hydrolysat enzymatique.

3. Composition cosmétique selon l'une quelconque des revendications précédentes, **caractérisée en ce que** la levure *Pichia naganishii* est obtenue à partir d'un exsudat de *Camellia japonica.*

4. Composition cosmétique selon l'une quelconque des revendications précédentes, **caractérisée en ce que** l'hydrolysat comprend entre 20% et 60% de protéines en poids de matière sèche de l'hydrolysat.

5. Composition cosmétique selon l'une quelconque des précédentes revendications, **caractérisée en ce que** l'hydrolysat est susceptible d'être obtenu par un procédé comprenant les étapes suivantes :
- solubilisation d'au moins 40 g/L de *Pichia naganishii* dans l'eau,
- hydrolyse enzymatique,
- séparation de la phase soluble et insoluble et récupération de la phase soluble,
- inactivation enzymatique, et
- éventuellement concentration et filtration stérilisante.

6. Composition cosmétique selon l'une quelconque des précédentes revendications, **caractérisée en ce qu'**elle comprend au moins 0,1% en poids de l'hydrolysat de *Pichia naganishii.*

7. Composition cosmétique selon l'une quelconque des revendications précédentes, **caractérisée en ce qu'**elle est adaptée à une application par voie topique

8. Utilisation cosmétique non thérapeutique d'un hydrolysat de *Pichia naganishii* ou de la composition selon l'une quelconque des précédentes revendications, pour améliorer l'hydratation et/ou protéger la peau humaine contre le dessèchement et/ou améliorer la fonction barrière.

9. Utilisation cosmétique non thérapeutique d'un hydrolysat de *Pichia naganishii* ou de la composition selon l'une quelconque des revendications 1 à 7, pour prévenir/réduire le vieillissement cutané.

## Patentansprüche

1. Kosmetische Zusammensetzung, die in einem physiologisch akzeptablen Medium mindestens ein Hydrolysat von *Pichia naganishii* umfasst.

2. Kosmetische Zusammensetzung nach dem vorhergehenden Anspruch, **dadurch gekennzeichnet, dass** das Hydrolysat ein enzymatisches Hydrolysat ist.

3. Kosmetische Zusammensetzung nach einem der vorhergehenden Ansprüche, **dadurch gekennzeichnet, dass** die Hefe *Pichia naganishii* aus einem Exsudat von *Camellia japonica* gewonnen wird.

4. Kosmetische Zusammensetzung nach einem der vorhergehenden Ansprüche, **dadurch gekennzeichnet, dass** das Hydrolysat zwischen 20 % und 60 % Protein, bezogen auf das Gewicht der Trockenmasse des Hydrolysats, umfasst.

5. Kosmetische Zusammensetzung nach einem der vorhergehenden Ansprüche, **dadurch gekennzeichnet, dass** das Hydrolysat durch ein Verfahren erhalten werden kann, das die folgenden Schritte umfasst:
- Solubilisierung von mindestens 40 g/L *Pichia naganishii* in Wasser,
- enzymatische Hydrolyse,
- Trennung der löslichen und unlöslichen Phase und Gewinnung der löslichen Phase,
- enzymatische Inaktivierung und
- gegebenenfalls Konzentrierung und sterilisierende Filtration.

6. Kosmetische Zusammensetzung nach einem der vorhergehenden Ansprüche, **dadurch gekennzeichnet, dass** sie mindestens 0,1 Gew.-% des Hydrolysats von *Pichia naganishii* umfasst.

7. Kosmetische Zusammensetzung nach einem der vorhergehenden Ansprüche, **dadurch gekennzeichnet, dass** sie für eine topische Anwendung geeignet ist.

8. Nicht-therapeutische kosmetische Verwendung eines Hydrolysats von *Pichia naganishii* oder der Zusammensetzung nach einem der vorhergehenden Ansprüche zur Verbesserung der Hydratation und/oder zum Schutz der menschlichen Haut vor Austrocknung und/oder zur Verbesserung der Barrierefunktion.

9. Nicht-therapeutische kosmetische Verwendung eines Hydrolysats von *Pichia naganishii* oder der Zusammensetzung nach einem der Ansprüche 1 bis 7, zur Vorbeugung/Verringerung der Hautalterung.

## Claims

1. Cosmetic composition comprising, in a physiologically acceptable medium, at least one *Pichia naganishii* hydrolysate.

2. Cosmetic composition according to the preceding claim, **characterized in that** the hydrolysate is an enzymatic hydrolysate.

3. Cosmetic composition according to any one of the preceding claims, **characterized in that** the yeast *Pichia naganishii* is obtained from an exudate of *Camellia japonica.*

4. Cosmetic composition according to any one of the preceding claims, **characterized in that** the hydrolysate comprises between 20% and 60% of proteins by weight of dry matter of the hydrolysate.

5. Cosmetic composition according to any one of the preceding claims, **characterized in that** the hydrolysate is obtainable by a process comprising the following steps:
- solubilization of at least 40 g/L of *Pichia naganishii* in water,
- enzymatic hydrolysis,
- separation of the soluble and insoluble phase and recovery of the soluble phase,
- enzymatic inactivation, and
- optionally concentration and sterilizing filtration.

6. Cosmetic composition according to any one of the preceding claims, **characterized in that** it comprises at least 0.1% by weight of the *Pichia naganishii* hydrolysate.

7. Cosmetic composition according to any one of the preceding claims, **characterized in that** it is suitable for topical application.

8. Non-therapeutic cosmetic use of a *Pichia naganishii* hydrolysate or of the composition according to any one of the preceding claims, for improving the moisturization and/or protecting human skin against drying out and/or improving the barrier function.

9. Non-therapeutic cosmetic use of a *Pichia naganishii* hydrolysate or of the composition according to any one of claims 1 to 7, for preventing/reducing skin ageing.
